# EUROPEAN PATENT SPECIFICATION

(11) **EP 3 269 338 B1**
(45) Date of publication and mention of the grant of the patent: **26.05.2021**
(21) Application number: 16179287.4
(22) Date of filing: 13.07.2016
(51) Int. Cl.: A61F 5/01, A61F 5/058

(54) **INFLATABLE BLADDER HAND ORTHOSIS**
AUFBLASBARE HANDORTHESE
ORTHÈSE À MAIN DE LA VESSIE GONFLABLE

(43) Date of publication of application: 17.01.2018
(73) Proprietor: MediRoyal Nordic AB, 192-07 Sollentuna (SE)
(72) Inventor: ERIKSSON, Thomas, SE-168 67 Bromma (SE)
(74) Representative: Barker Brettell Sweden AB

(56) References cited:
- US-A- 4 977 890
- US-A- 5 437 620
- US-A- 5 916 186

## Description

### TECHNICAL FIELD

The present embodiments generally relate to hand orthoses, and in particular to inflatable bladder hand orthoses.

### BACKGROUND

Orthoses are externally applied devices that can be used to modify or support structural and/or functional characteristics of the neuromuscular and skeletal system. Orthoses may be used in various applications to control, guide, limit or immobilize an extremity, a joint and/or a body segment of a human subject.

For instance, U.S. patent no. 5,020,515 discloses an inflatable hand splint for treatment of human subjects with arthritic or stroke paralyzed writs. The inflatable hand splint is disclosed as being easily applied under the subject's fingers between the finger tips and the palmer region of the hand. By inflating the device, the fingers are gently moved outward from the palmer region repeatedly on a scheduled basis to prevent fixation of the fingers in a contorted position.

U.S. patent no. 5,056,504 discloses an inflatable ball hand splint comprising a pliable wrist element with an inflatable ball attached at a first end. The second end of the wrist element extends downwardly along the underside of the subject's wrist and is strapped to the subject's forearm with a pair of straps. A front end of a hard plastic support member is inserted between the fingers and the palmer region of the subject's hand. The rear end of the support member is located below the pliable wrist element and is also strapped to the subject's forearm.

U.S. patent no. 5,437,620 discloses a wrist splint comprising a splint platform, at least one bladder receptacle member slidingly engaged with the splint platform and at least one air bladder. Each air bladder is coupled to one of the bladder receptacle members.

There is, however, still a need for improvements within the technical field of inflatable hand orthoses and in particular for human subjects having spasticity and/or contractures in the wrist, hand and/or digits, such as following a neurological disease or cerebrovascular accident (CVA).

### SUMMARY

It is a general objective to provide an improved inflatable bladder hand orthosis.

It is a particular objective to provide an inflatable bladder hand orthosis useful for human subjects having spasticity and/or contractures following a neurological disease or CVA.

These and other objectives are met by embodiments disclosed herein.

An aspect of the embodiments relates to an inflatable bladder hand orthosis comprising a splint, an inflatable bladder and a bladder support. The splint comprises a palmar splint section adapted to be aligned with at least a portion of a palm of a hand of a human subject and a wrist and forearm splint section integral with or connected to the palmar splint section. The wrist and forearm splint section extends from the palmar splint section to a distance corresponding to at least a portion of a forearm of the human subject. The inflatable bladder is adapted to be aligned with metacarpophalangeal (MCP) joints of the fingers of the hand and is inflatable by introduction of a fluid within an interior chamber of the inflatable bladder. The bladder support is pivotally connected to the palmar splint section and comprises a bladder connecting surface opposite to a surface of the bladder support facing the palmar splint section. The inflatable bladder is arranged on the bladder connecting surface.

The inflatable bladder hand orthosis is, in particular, suitable for human subjects with ulnar or radial deviation. The inflatable bladder hand orthosis provides a dynamic extension of the flexor tendons on human subjects with spasticity. The inflatable bladder hand orthosis supports the MCP arch and the thumb in volar abduction, thereby providing a dynamic extension that assists in reducing tonus and strive to place the hand in a reflex inhibitory position.

### BRIEF DESCRIPTION OF THE DRAWINGS

The embodiments, together with further objects and advantages thereof, may best be understood by making reference to the following description taken together with the accompanying drawings, in which:
Fig. 1 illustrates a splint and a bladder support of an inflatable bladder hand orthosis according to an embodiment as seen from a first main side;
Figs. 2A and 2B illustrate the splint and bladder support of Fig. 1 with the bladder support pivoted relative to the splint as seen from the first main side;
Fig. 3 illustrates the splint of Fig. 1 as seen from a second, opposite main side;
Fig. 4 illustrates the splint and bladder support of Fig. 1 as seen from a front side;
Fig. 5 illustrates a bladder support according to an embodiment;
Fig. 6 illustrates a portion of the splint and bladder support of Fig. 1 with bladder support pivoted relative to the splint as seen from the second, opposite main side;
Fig. 7 illustrates an inflatable bladder of the inflatable bladder hand orthosis according to an embodiment;
Fig. 8 illustrates an inflatable bladder of the inflatable bladder hand orthosis according to another embodiment;
Fig. 9 illustrates an inflatable bladder hand orthosis with an inflatable bladder attached to the bladder support according to an embodiment; and
Fig. 10 illustrates a textile dress that can be used in the inflatable bladder hand orthosis according to an embodiment.

### DETAILED DESCRIPTION

Throughout the drawings, the same reference numbers are used for similar or corresponding elements.

The present embodiments generally relate to hand orthoses, sometimes referred to as wrist-hand-finger orthoses (WHFO) or wrist-hand orthoses within the technical field. In particular, the present embodiments relate to inflatable bladder hand orthoses that are particular useful for human subjects having spasticity and/or contractures in the wrist, hand and/or digits. Such medical conditions, i.e. spasticity and/or contractures, may occur in subjects having suffered from a neurological disease or cerebrovascular accident (CVA), also referred to as cerebrovascular insult (CVI) or simply stroke.

For instance, CVA victims may suffer from a spastic pattern generally referred to as flexion pattern with anteriorly rotated shoulder, i.e. shoulder subluxation, flexed elbow joint, flexed wrist and bent fingers.

An inflatable bladder hand orthosis of the embodiments may be beneficial to such subjects by preventing or reducing spasticity, achieving tonus reduction and preventing or reducing shortening of the flexor tendon to increase the passive range of motion and stretch the contracture.

The inflatable hand splints as disclosed in U.S. patent nos. 5,020,515 and 5,056,504 may be useful for stroke victims and other subjects suffering from hand paralysis to unbend severely contorted fingers. However, several such subjects may in addition suffer from radial deviation, or more commonly ulnar deviation of the metacarpophalangeal (MCP) joints. Such ulnar deviation, also referred to as ulnar drift, is caused by shortening of the flexor tendons, especially the 4^{th} and 5^{th} flexor tendons. This causes the fingers and the middle hand to drift towards the ulnar side, i.e. the little finger side. Radial deviation, also referred to as radial drift, correspondingly causes the fingers and the middle hand to drift towards the radial side.

The ulnar or radial drift may in fact cause the air bladder of the inflatable hand splints in U.S. patent nos. 5,020,515 and 5,056,504 to come loose from the hard plastic or the wrist element, respectively. Hence, such prior art inflatable hand splints have shortcomings, in particular with regard to human subjects suffering from ulnar or radial deviation.

An aspect of the embodiments relates to an inflatable bladder hand orthosis. The inflatable bladder hand orthosis comprises a splint, an inflatable bladder and a bladder support. The splint comprises a palmar splint section adapted to be aligned with at least a portion of a palm of a hand of a human subject. The splint also comprises a wrist and forearm splint section integral with or connected to the palmar splint section. The wrist and forearm splint section extends from the palmar splint section to a distance corresponding to at least a portion of a forearm of the human subject. The inflatable bladder of the inflatable bladder hand orthosis is adapted to be aligned with MCP joints of the fingers of the hand. The inflatable bladder is inflatable by introduction of a fluid within an interior chamber of the inflatable bladder. The bladder support is pivotally connected to the palmar splint section and comprises a bladder connecting surface opposite to a surface of the bladder support facing the palmar splint section. The inflatable bladder is then arranged on the bladder connecting surface of the bladder support.

Hence, the inflatable bladder hand orthosis comprises a pivotal bladder support that interconnects the inflatable bladder to the splint. This means that the bladder support, and thereby the inflatable bladder, is pivotal relative to the palmar splint section and the splint. This pivotal arrangement of the inflatable bladder relative to the splint by means of the pivotally connected bladder support implies that the inflatable bladder hand orthosis can compensate for any ulnar or radial deviation with a significantly reduced risk of the inflatable bladder coming loose.

A further significant advantage of having the inflatable bladder pivotally connected to the splint by means of the bladder support is that is possible to optimize and adjust the position of the inflatable bladder relative to the hand of the particular subject. In particular, the inflatable bladder can accurately be adjusted not only along a distal-to-proximal axis but also pivotally adjusted to be positioned aligned with the MCP joints of the hand even if the fingers and the middle hand are drifted towards the ulnar or radial side. Such a pivotal adjustment of the inflatable bladder relative to the splint is not possible in the prior art inflatable hand orthoses, such as represented by U.S. patent nos. 5,020,515 and 5,056,504.

Various embodiments of the inflatable bladder hand orthosis will now be further described with reference to the drawings.

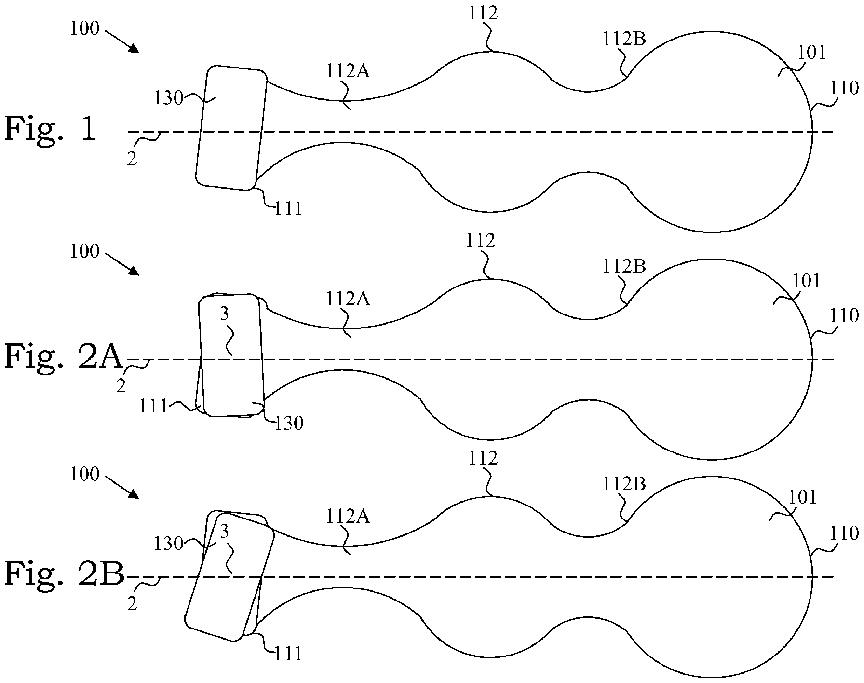

Fig. 1 illustrates a splint 110 and a bladder support 130 of an inflatable bladder hand orthosis 100 according to an embodiment as seen from a first main side 101. Figs. 2A and 2B illustrate the splint 110 and the bladder support 130 of Fig. 1 with the bladder support 130 pivoted relative to the splint 110 in the radial direction (Fig. 2A) or in the ulnar direction (Fig. 2B).

The splint 110 comprises the palmar splint section 111 adapted to be aligned with at least a portion of the palm of the hand of a human subject. The splint 110 also comprises the wrist and forearm splint section 112. This wrist and forearm splint section 112 may be integral with the palmar splint section 111 as shown in the figures. In such an embodiment, the splint 110 is an integral piece comprising the palmar and wrist and forearm splint sections 111, 112.

In another embodiment, the wrist and forearm splint section 112 is connected to the palmar splint section 111. In such an embodiment, these two splint sections 111, 112 are two separate pieces that are connected or attached to each other.

The wrist and forearm splint section 112 comprises, in the illustrated embodiment, a forearm portion 112B to be aligned with human subject's forearm and a wrist portion 112A interconnecting the forearm portion 112B and the palmar splint section 111.

In an embodiment, the wrist portion 112A not only interconnects the forearm portion 112B of the wrist and forearm splint section 112 and the palmar splint section 111 but is preferably deformable in at least a portion adapted to be aligned with the wrist joint of the human subject's hand. In such a case, the angle between the forearm portion 112B and the palmar splint section 111 can be adapted to a suitable angle of the human subject's wrist.

For instance, the wrist portion 112A may be adapted to support the wrist in 0° to 20° extension relative to the back of the hand (dorsal extension).

In other embodiments, the wrist and forearm splint section 112 is made of a rigid or at least semi-rigid material to present a 0° angle in the wrist or a pre-set, i.e. fixed, non-zero angle in the wrist. In such a case, there is no need for any deformable materials of the splint 110, of the wrist and forearm splint section 112 or at least of the wrist portion 112A of the wrist and forearm splint section 112.

The palmar splint section 111 is, as mentioned above, designed to be aligned with at least a portion of the palm of the human subject's hand. Accordingly, the size and form of this palmar splint section 111 is preferably selected to be able to fit at least a portion of the palm.

The wrist and forearm splint section 112 can have any general form or size as long as it is capable of extending from the palmar splint section 111 along a distal-to-proximal axis 2 to a distance corresponding to at least a portion of the human subject's forearm.

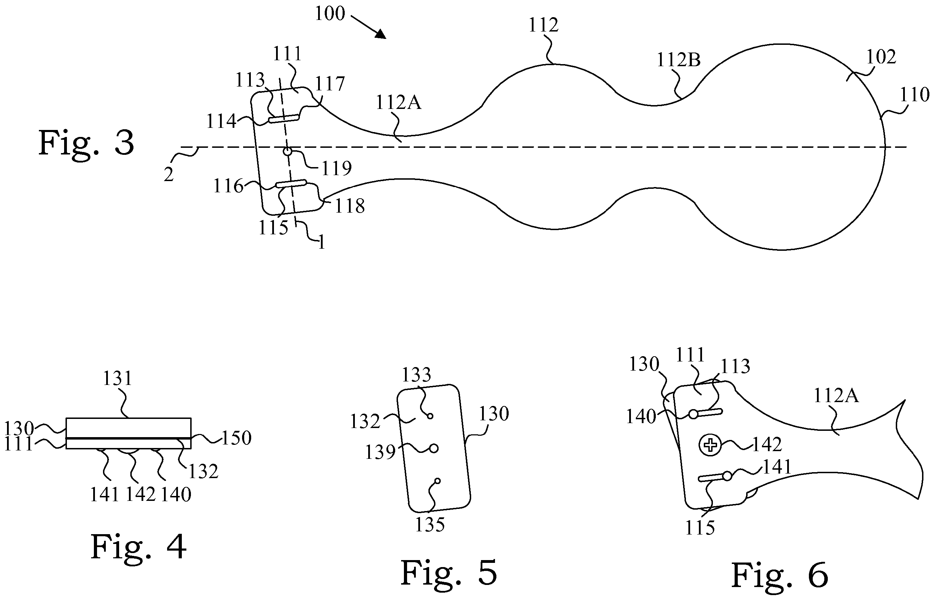

For instance, the splint 110 can be manufactured in the form of a shaped sheet of, for instance, a plastic or metal material. A further variant is a palmar splint section 111 in the form of a shaped sheet and with the wrist and forearm splint section 112 in the form of a wire-based open frame structure. The wrist and forearm splint section 112 could then be constructed as a wire, for instance a plastic or metal wire, extending longitudinally, i.e. along the distal-to-proximal axis 2, and then turning back to form an open frame structure or loop. The wire preferably extends back to the palmar splint section 111, where the two ends of the wire are attached to the palmar splint section 111. In such a case, the longitudinal portions of the wire extending substantially along the distal-to-proximal axis 2 will run along a portion of the length of the human subject's forearm. Correspondingly, the transversal portions extending substantially along or angled (diagonally) relative to a radial-to-ulnar axis 1 (see Fig. 3) extend over the underside or caudal side of the forearm.

In Fig. 1, the bladder support 130 is aligned with the palmar splint section 111. Fig. 2A illustrates a situation in which the bladder support 130 is pivoted relative to the palmar splint section 111 around a pivotal point 3 in a radial direction. Fig. 2B correspondingly illustrates the bladder support 130 pivoted relative to the palmar splint section 111 around the pivotal pint 3 in the ulnar direction.

In an embodiment, the bladder support 130 is pivotal relative to the palmar splint section 111 around the pivotal point 3 between a radial end pivotal position and an ulnar end pivotal position.

In this embodiment, the bladder support 130 can thereby pivot or rotate relative to the palmar splint section 111 clock-wise or counter clock-wise around the pivotal point 3 to a respective end pivotal position, i.e. the radial end pivotal position and the ulnar end pivotal position.

This embodiment is described in more detail with reference to Figs. 3 to 6. Fig. 3 illustrates the splint 110 of Fig. 1 as seen from a second, opposite main side 102. In an embodiment, the palmar splint section 111 comprises a slot 113, 115 arranged along a radial-to-ulnar axis 1 passing through the pivotal point 3. The bladder support 130 then comprises, see Fig. 5, a threaded blind hole 133, 135 aligned with at least a portion of the slot 113,115. In this embodiment, the inflatable bladder hand orthosis 100 comprises a screw 140, 141 passing though the slot 113, 115 (see Fig. 6) and entering the threaded blind hole 133, 135 (see Fig. 4). A first end 114, 116 of the slot 113, 115 is adapted to engage a shank of the screw 140, 141 when the bladder support 130 is in the radial end pivotal position relative to the palmar splint section 111 as shown in Fig. 2A. Correspondingly, a second, opposite end 117, 118 of the slot 113, 115 is adapted to engage the shank when the bladder support 130 is in the ulnar end pivotal position relative to the palmar splint section 111 as shown in Fig. 2B.

In the above described embodiment, the pivotal or rotation limitation is achieved by means of at least one screw 140, 141 running in a respective slot 113, 115 in the palmar splint section 111 and attached or anchored in the bladder support 130 via a respective threaded blind hole 133, 135. This in turn implies that the bladder support 130 can be pivoted or rotated relative to the palmar splint section 111 and the splint 110 around the pivotal point 3 between end pivotal positions in which the shank of the screw 140, 141 engages either end 114, 117; 116, 118 of the slot 113, 115. The length of the slot 113, 115 in the palmar splint section 111 thereby dictates or restricts how far the bladder support 130 can be pivoted or rotated around the pivotal point 3. Thus, the slot length defines the maximum pivotal or rotation angle of the bladder support 130 around the pivotal point 3 and relative to the palmar splint section 111.

In an embodiment, the palmar splint section 111 comprises a single slot 113 and the bladder support 130 comprises a single matching threaded blind hole 133. In such a case, the slot 113 could be arranged on either the radial or ulnar side along the radial-to-ulnar axis 1 and relative to the pivotal point 3, and thereby relative to the distal-to-proximal axis 2.

In another embodiment, two slots 113, 115, two threaded blind holes 133,135 and two screws 140, 141 are used as shown in Figs. 3 to 6. In such an embodiment, the palmar splint section 111 comprises a first slot 113 arranged along the radial-to-ulnar axis 1 on a radial side relative to the pivotal point 3 and a second slot 115 arranged along the radial-to-ulnar axis 1 on an ulnar side relative to the pivotal point 3. The bladder support 130 comprises a first threaded blind hole 133 aligned with at least a portion of the first slot 113 and a second threaded blind hole 135 aligned with at least a portion of the second slot 115. The inflatable bladder hand orthosis 100 comprises a first screw 140 passing through the first slot 113 and entering the first threaded blind hole 133 and a second screw 141 passing through the second slot 115 and entering the second threaded blind hole 135.

In this embodiment, a first end 114 of the first slot 113 is adapted to engage a shank of the first screw 140 and a second end 118 of the second slot 115 is adapted to engage a shank of the second screw 141 when the bladder support 130 is in the radial end pivotal position relative to the palmar splint section 111. Correspondingly, a second, opposite end 117 of the first slot 113 is adapted to engage the shank of the first screw 140 and a first, opposite end 116 of the second slot 115 is adapted to engage the shank of the second screw 141 when the bladder support 130 is in the ulnar end pivotal position relative to the palmar splint section 111.

In an embodiment, the one or two screws 140,141 are merely used to restrict and define, when engaging either end 114, 117; 116, 118 of the one or two slots 113, 115, the radial and ulnar end pivotal positions of the bladder support 130 relative to the palmar splint section 111. In such an embodiment, the bladder support 130 may freely, i.e. without any or merely limited resistance or friction, pivot or rotate around the pivotal point 3. In such an embodiment, the length of the shank(s) of the screw(s) 140, 141 is(are) preferably slightly longer than the depth of the threaded blind hole(s) 133, 135 plus the thickness of the palmar splint section 111. Hence, there is preferably a small distance or gap between the underside of the head(s) of the screw(s) 140, 141 and the second main surface 102 of the palmar splint section 111 when the screw(s) 140, 141 is(are) screwed to the bottom of the threaded blind hole(s) 133, 135.

In another embodiment, the screw(s) 140, 141 can be used to tighten or reversibly lock the bladder support 130 to the palmar splint section 111. In this embodiment, the length of shank(s) of the screw(s) 140, 141 is(are) preferably equal to or slightly shorter than the depth of the threaded blind hole(s) 133, 135 plus the thickness of the palmar splint section 111. Tightening the screw(s) thereby brings the surface 132 of the bladder support 130 facing the palmar splint section 111 and the first main surface 101 (see Figs. 1, 2A, 2B) tight together thereby increasing the friction therebetween.

As a consequence, the pivotal position of the bladder support 130 relative to the palmar splint section 111 and the splint 110 can be reversibly locked by screwing the screw(s) tight into the threaded blind hole(s) 133, 135. This means that the bladder support 130 and thereby the inflatable bladder supported by the bladder support 130 can be set to a desired pivotal position adapted to the human subject's hand and MCP joints to thereby adjust to any ulnar or radial deviation.

It may be preferred that this reversible locking by bringing the surfaces 132, 101 tight together can be released or overcome if the human subject's hand suddenly should drift towards the radial or ulnar side. In such a case, the bladder support 130 is thereby released from its pivotal lock to the palmar splint section 111 rather than causing the inflatable bladder 120 to come loose from the bladder support 130.

In an embodiment and as best shown in Fig. 4, the inflatable bladder hand orthosis 100 may comprise a sliding sheet 150 arranged between the palmar splint section 111 and the bladder support 130 to facilitate pivotal of the bladder support 130 relative to the palmar splint section 111.

This sliding sheet 150 may thereby be arranged intermediate the bladder support 130 and the palmar splint section 111 to reduce any friction therebetween, i.e. between the surface 132 of the bladder support 130 and the first main surface 101 of the palmar splint section 111.

Such a sliding sheet 150 is also compatible with the above described embodiment using the screw(s) 140, 141 to reversibly lock bladder support 130 in a selected pivotal position relative the palmar splint section 111.

The sliding sheet 150 may be made of any material that would reduce the friction between the bladder support 130 and the palmar splint section 111. Non-limiting examples of such materials include polytetrafluoroethylene (PTFE), such as TEFLON®, polyethylene and polypropylene.

In an embodiment, the palmar splint section 111 comprises a through hole 119 extending through a thickness of the palmar splint section 111. The bladder support 130 then comprises a threaded blind hole 139 aligned with the through hole 119. The inflatable bladder hand orthosis 100 comprises a screw 142 passing through the through hole 119 and entering the threaded blind hole 139 to pivotally connect the bladder support 130 to the palmar splint section 111.

Hence, in this embodiment the pivotal connection between the bladder support 130 and the palmar splint section 111 is achieved by a screw 142, a though hole 119 and a threaded blind hole 139. The pivotal point 3 previously mentioned herein thereby corresponds to the position of the through hole 119 (and of the screw 142 and the threaded blind hole 139).

Other technologies of pivotally connecting the bladder support 130 to the palmar splint section 111 could alternatively be used and the invention is not limited to the above described embodiment.

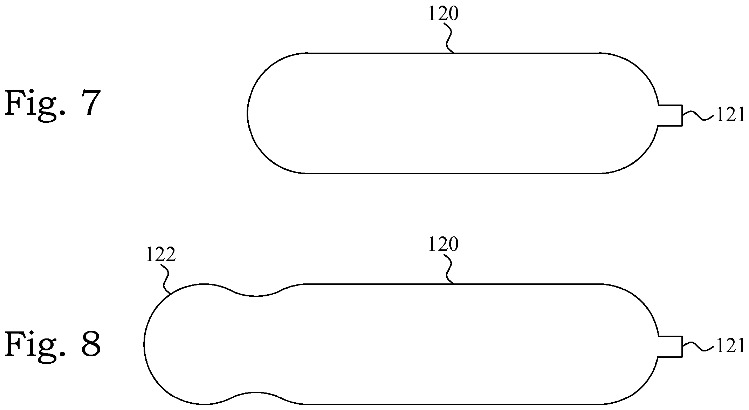

Figs. 7 and 8 illustrate inflatable bladders 120 that could be used in the inflatable bladder hand orthosis 100 of the embodiments.

In Fig. 7, the inflatable bladder 120 is, in an inflated state, in the form of a sausage with a fluid valve integral with the inflatable bladder 120 and preferably arranged in connection with one of its short ends. The inflatable bladder 120 is preferably elongated along the radial-to-ulnar axis 2 and has a general "sausage-like" shape when inflated. This shape of the inflatable bladder 120 is effectively adapted to and supports the MCP joints of the fingers.

The inflatable bladder 120 is inflatable by introduction of a fluid within an interior chamber of the inflatable bladder 120. The fluid could, for instance, be a gas, including a gas mixture, or a liquid. The fluid is preferably air. In a deflated state, the inflatable bladder 120 is positioned substantially flat on the bladder connecting surface 131 of the bladder support 130. However, once the fluid, such as air, is being introduced, typically pumped, into the interior chamber of the inflatable bladder 120, the inflatable bladder 120 starts to expand and extend from the surface 131.

Various means for attaching the inflatable bladder 120 to the bladder connecting surface 131 of the bladder support 130 are possible, for instance gluing, welding, etc. depending on the particular materials of the inflatable bladder 120 and the bladder support 130.

The introduction of fluid into the interior chamber of the inflatable bladder 130 causes an upper or dorsal portion of the inflatable bladder 120 to rise from the bladder support surface 131, while the lower or palmar portion of the inflatable bladder 120 is preferably still attached to the bladder support surface 131.

The palmar splint section 111 of the inflatable bladder hand orthosis 100 and its attached or connected inflatable bladder 120 will, by inflating the inflatable bladder 120, provide an individually adjustable support to the MCP joints and fingers of the human subject's hand together with support of the wrist on the wrist and forearm splint section 112. A pump is connectable to the valve 121 of the inflatable bladder 120 to introduce fluid, such as air, into the first inflatable bladder 120 to a suitable level depending on the human subject's needs and medical condition. The level of inflation of the inflatable bladder 120 can be adjusted gradually to follow the progress of the human subject. The inflatable bladder 120 can thereby be used to open up the hand and to provide pressure absorption and finger extension.

In this way, the inflatable bladder hand orthosis 100 can be used for reduction of spasticity and/or to prevent or stretch contractures in the fingers and wrist, such after a neurological disease or CVA.

The valve 121 is opened when a pump is connected to the valve 121 and presses fluid, such as air, into the interior chamber of the inflatable bladder 120. Correspondingly, when the fluid flow into the interior chamber stops the valve 121 preferably closes to thereby prevent fluid from escaping from the inflatable bladder 120. In a particular embodiment, the pump or another equipment can also be used to deflate the inflatable bladder 120 by sucking or draining fluid out from the inflatable bladder 120 through the valve 121.

In a simpler embodiment, a plug is inserted into an opening into the interior chamber of the inflatable bladder 120 following inflating the inflatable bladder 120 by the pump to prevent the fluid from escaping. When the inflatable bladder 120 is to be deflated, the plug is simply removed allowing fluid to escape from the inflatable bladder 120. This embodiment provides an easier construction relaxing the need for a valve 121. However, it generally leads to less control of the inflation level of the inflatable bladder 120.

The inflatable bladder 120 as shown in Fig. 7 gives an efficient support of the MCP arch and the thumb in volar abduction. Fig. 8 illustrates another embodiment of an inflatable bladder 120. In this embodiment, the inflatable bladder 120 has, as the embodiment shown in Fig. 7, a general sausage-like shape but with a waist forming a ball-like end structure 122. The waist-formed inflatable bladder 120 as shown in Fig. 8 provides support to the thenar part of the thumb, which may be advantageous in order to maintain a ring grip. This will in turn prevent contractures on the adduction muscles in the thumb.

The designs of the inflatable bladder 120 as shown in Figs. 7 and 8 should merely be seen as illustrative design options. There are other possible variants. For instance, the inflatable bladder could be conically shaped with a larger diameter on the metacarpal 4 and 5. Such a conical inflatable bladder could have a positive effect on releasing tonus. If the fingers of the human subject are really contracted, a larger cylinder shaped bladder could be difficult to fit inside the hand, due to the contractures of the fingers or the increased tonus.

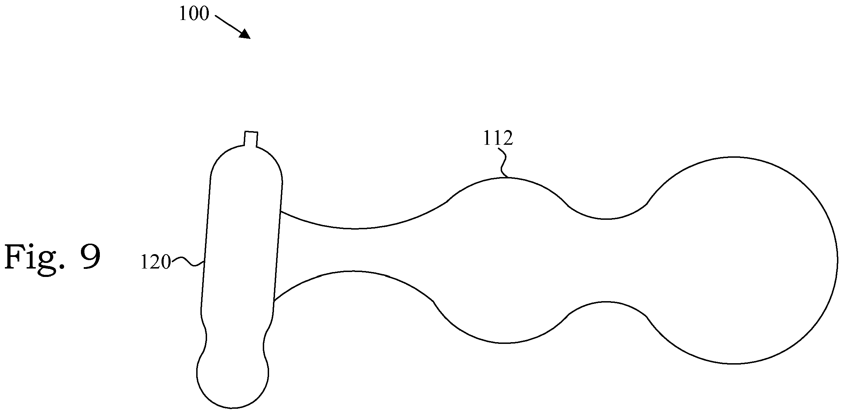

Fig. 9 illustrates an inflatable bladder hand orthosis 100 of the embodiments with the inflatable bladder 120 attached to the bladder support 130.

The inflatable bladder hand orthosis 100 of the embodiments provides a dynamic extension of the flexor tendons on human subjects with spasticity. The support of the MCP arch and the thumb in volar abduction provides a dynamic extension, which assists in reducing tonus and striving to place the hand in a reflex inhibitory position (RIP).

The splint 110 of the inflatable bladder hand orthosis 100 is preferably manufactured from a metal material, including a metal alloy material. Suitable, but non-limiting examples of such metal (alloy) materials include iron alloys, aluminum and aluminum alloys, e.g. malleable aluminum or aluminum alloys.

The metal material of the splint 110 is preferably deformable at least in the wrist portion 112A of the wrist and forearm splint section 112. Hence, the metal material is preferably not brittle and can be bent slightly to form a bend at the wrist portion 112A that are adapted to the particular human subject.

Although metal materials may be suitable for manufacturing the splint 110 other materials could alternatively be used, such as plastics and in particular low temperature thermoplastics, e.g. such low temperature thermoplastics that are heated in hot water at, for instance, 75 to 85°C or using at heat gun.

The bladder support 130 of the inflatable bladder hand orthosis 100 may be manufactured from metal or plastic materials similar to the splint 110.

In an optional embodiment, the first main surface 101 of the splint 110 facing the wrist and forearm of the patient and/or the second, opposite main surface 102 of the splint 110 may be coated with or comprise a pressure reducing coating, layer or laminate. Such a pressure reducing coating, layer or laminate will increase the comfort for the patient wearing the inflatable bladder hand orthosis 100. If both main surfaces 101,102 of the splint 110 comprise a respective pressure reducing coating, layer or laminate, a sandwich structure is obtained.

Any pressure reducing materials known in the art and that can be coated onto or otherwise applied to the splint 110 could be used according to the embodiments. Non-limiting examples include polychloroprene materials, such as NEOPRENE®. In particular, NEOPRENE® coatings, layers or laminates with closed cells could be used as pressure reducing material.

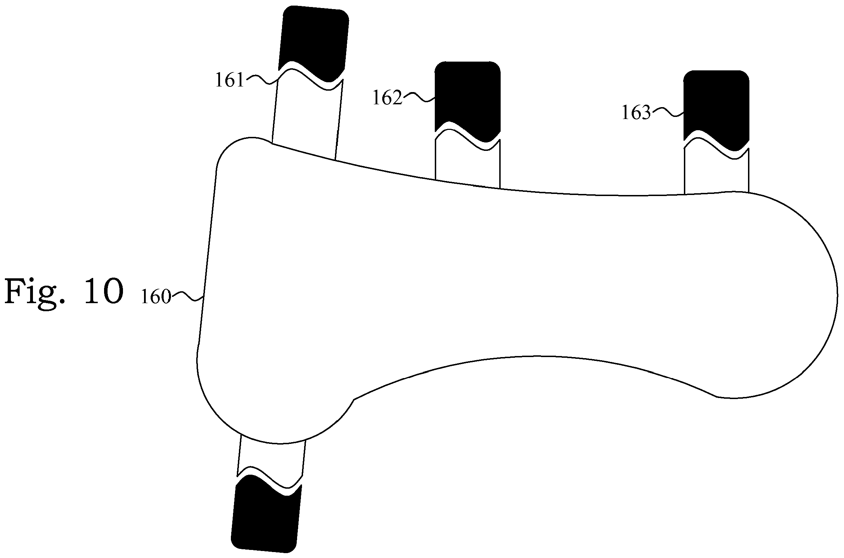

The inflatable bladder hand orthosis 100 may comprises a textile dress 160, also referred as cloth dress, see Fig. 10. The textile dress 160 has an interior chamber adapted to house the splint 110, the bladder support 130 and the inflatable bladder 120. The textile dress 160 comprises multiple closing straps 161, 162, 163 arranged along a distal-to-proximal axis 2 of the inflatable bladder hand orthosis 100. In such a case, at least one closing strap 161 is arranged at a portion of the textile dress 160 aligned with the palmar splint section 111 when the splint 110 is housed within the interior chamber of the textile dress 160. Furthermore, at least one closing strap 162, 163 is arranged at a portion of the textile dress 160 aligned with the wrist and forearm splint section 112 when the splint 110 is housed within the interior chamber of the textile dress 160. The multiple closing straps 161, 162, 163 are arranged to secure the inflatable bladder hand orthosis 100 to the hand and forearm of the human subject.

The textile dress 160, thus, has dual functions for the inflatable bladder hand orthosis 100. Firstly, it secures the inflatable bladder hand orthosis 100 to the hand and forearm of the human subject using the closing straps or bands 161, 162, 163 that are wrapped around the hand and the forearm. The closing straps 161, 162, 163 are preferably secured at the textile dress 160 by means of hook and loop fasteners, such as VELCRO® fasteners. In such a case, at least a portion of the closing straps 161, 162, 163 comprises hook or loop material as schematically shown in Fig. 10. The hook or loop material can then mate with loop or hook material provided on the textile dress 160 or on the opposite side of the closing straps 161, 162, 163. The mating between the loop and hook material is non-permanent implying that the closing straps 161, 162, 163 may be opened and closed when removing and attaching the inflatable bladder hand orthosis 100 to a subject, respectively.

Secondly, the textile dress 160 constitutes a soft surface facing the skin of the hand and forearm. The textile dress 160 could thereby provide pressure absorption to make the inflatable bladder hand orthosis 100 comfortable to wear.

Various textile materials can be used, including filled textile materials.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

## Claims

1. An inflatable bladder hand orthosis (100) comprising:
a splint (110) comprising:
a palmar splint section (111) adapted to be aligned with at least a portion of a palm of a hand of a human subject; and
a wrist and forearm splint section (112) integral with or connected to said palmar splint section (111), said wrist and forearm splint section (112) extending from said palmar splint section (111) to a distance corresponding to at least a portion of a forearm of said human subject; and
an inflatable bladder (120) adapted to be aligned with metacarpophalangeal, MCP, joints of the fingers of said hand, said inflatable bladder (120) being inflatable by introduction of a fluid within an interior chamber of said inflatable bladder (120),
a bladder support (130) connected to said palmar splint section (111) and comprising a bladder connecting surface (131) opposite to a surface (132) of said bladder support (130) facing said palmar splint section (111); and
said inflatable bladder (120) is arranged on said bladder connecting surface (131),
**characterized in that**
the bladder support is pivotally connected to the palmar splint section.

2. The inflatable bladder hand orthosis according to claim 1, **characterized in that** said bladder support (130) is pivotal relative to said palmar splint section (111) around a pivotal point (3) between a radial end pivotal position and an ulnar end pivotal position.

3. The inflatable bladder hand orthosis according to claim 2, **characterized in that**
said palmar splint section (111) comprises a slot (113, 115) arranged along a radial-to-ulnar axis (1) passing through said pivotal point (3);
said bladder support (130) comprises a threaded blind hole (133, 135) aligned with at least a portion of said slot (113, 115);
said inflatable bladder hand orthosis (100) comprises a screw (140, 141) passing through said slot (113, 115) and entering said threaded blind hole (133, 135);
a first end (114, 116) of said slot (113, 115) is adapted to engage a shank of said screw (140, 141) when said bladder support (130) is in said radial end pivotal position relative to said palmar splint section (111); and
a second, opposite end (117, 118) of said slot (113, 115) is adapted to engage said shank when said bladder support (130) is in said ulnar end pivotal position relative to said palmar splint section (111).

4. The inflatable bladder hand orthosis according to claim 3, **characterized in that** said palmar splint section (111) comprises:
a first slot (113) arranged along said radial-to-ulnar axis (1) on a radial side relative to said pivotal point (3); and
a second slot (115) arranged along said radial-to-ulnar axis (1) on an ulnar side relative to said pivotal point (3);
said bladder support (130) comprises:
a first threaded blind hole (133) aligned with at least a portion of said first slot (113); and
a second threaded blind hole (135) aligned with at least a portion of said second slot (115);
said inflatable bladder hand orthosis (100) comprises:
a first screw (140) passing through said first slot (113) and entering said first threaded blind hole (133); and
a second screw (141) passing through said second slot (133) and entering said second threaded blind hole (135);
a first end (114) of said first slot (113) is adapted to engage a shank of said first screw (140) and a second end (118) of said second slot (115) is adapted to engage a shank of said second screw (141) when said bladder support (130) is in said radial end pivotal position relative to said palmar splint section (111); and
a second, opposite end (117) of said first slot (113) is adapted to engage said shank of said first screw (140) and a first, opposite end (116) of said second slot (115) is adapted to engage said shank of said second screw (141) when said bladder support (130) is in said ulnar end pivotal position relative to said palmar splint section (111).

5. The inflatable bladder hand orthosis according to any of the claims 1 to 4, **characterized by** a sliding sheet (150) arranged between said palmar splint section (111) and said bladder support (130) to facilitate pivotal of said bladder support (130) relative to said palmar splint section (111).

6. The inflatable bladder hand orthosis according to any of the claims 1 to 5, **characterized in that**
said palmar splint section (111) comprises a through hole (119) extending through a thickness of said palmar splint section (111);
said bladder support (130) comprises a threaded blind hole (139) aligned with said through hole (119); and
said inflatable bladder hand orthosis (100) further comprises a screw (142) passing through said through hole (119) and entering said threaded blind hole (139) to pivotally connect said bladder support (130) to said palmar splint section (111).

7. The inflatable bladder hand orthosis according to any of the claims 1 to 6, **characterized in that** said wrist and forearm splint section (112) comprises:
a forearm portion (112B) to be aligned with said forearm; and
a wrist portion (112A) interconnecting said forearm portion (112B) and said palmar splint section (111), said wrist portion (112A) is deformable at least in a portion adapted to be aligned with the wrist joint of said hand.

8. The inflatable bladder hand orthosis according to any of the claims 1 to 7, **characterized by** a textile dress (160) having an interior chamber adapted to house said splint (110), said bladder support (130) and said inflatable bladder (120), said textile dress (160) comprising multiple closing straps (161, 162, 163) arranged along a distal-to-proximal axis (2) of said inflatable bladder hand orthosis (100) with at least one closing strap (161) arranged at a portion of said textile dress (160) aligned with said palmar splint section (111) when said splint (110) is housed within said interior chamber of said textile dress (160) and at least one closing strap (162, 163) arranged at a portion of said textile dress (160) aligned with said wrist and forearm splint section (112) when said splint (110) is housed within said interior chamber of said textile dress (160), said multiple closing straps (161, 162, 163) are arranged to secure said inflatable bladder hand orthosis (100) to said hand and said forearm.

## Patentansprüche

1. Handorthese (100) mit aufblasbarer Blase, umfassend:
eine Schiene (110), umfassend:
einen palmaren Schienenabschnitt (111), der geeignet ist, um auf mindestens einen Teil einer Handfläche einer Hand einer Versuchsperson ausgerichtet zu sein; und
einen Handwurzel- und Unterarm-Schienenabschnitt (112), der mit dem palmaren Schienenabschnitt (111) einstückig oder verbunden ist, wobei sich der Handwurzel- und Unterarm-Schienenabschnitt (112) von dem palmaren Schienenabschnitt (111) aus über eine Distanz, die mindestens einem Teil eines Unterarms der Versuchsperson entspricht, erstreckt; und
eine aufblasbare Blase (120), die geeignet ist, um auf Metakarpophalangeal-, MCP, Gelenke der Finger der Hand ausgerichtet zu sein, wobei die aufblasbare Blase (120) durch die Einführung eines Fluids in eine Innenkammer der aufblasbaren Blase (120) aufblasbar ist,
einen Blasenträger (130), der mit dem palmaren Schienenabschnitt (111) verbunden ist und eine Blasenverbindungsfläche (131) gegenüber einer Oberfläche (132) des Blasenträgers (130), die dem palmaren Schienenabschnitt (111) gegenübersteht, umfasst; und
wobei die aufblasbare Blase (120) auf der Blasenverbindungsfläche (131) angeordnet ist,
**dadurch gekennzeichnet, dass** der Blasenträger mit dem palmaren Schienenabschnitt schwenkbar verbunden ist.

2. Handorthese mit aufblasbarer Blase nach Anspruch 1, **dadurch gekennzeichnet, dass** der Blasenträger (130) im Verhältnis zu dem palmaren Schienenabschnitt (111) um einen Schwenkpunkt (3) zwischen einer radialen Endschwenkposition und einer ulnaren Endschwenkposition schwenkbar ist.

3. Handorthese mit aufblasbarer Blase nach Anspruch 2, **dadurch gekennzeichnet, dass**
der palmare Schienenabschnitt (111) ein Langloch (113, 115) umfasst, das entlang einer radialen-ulnaren Achse (1), die durch den Schwenkpunkt (3) geht, angeordnet ist;
der Blasenträger (130) ein Gewindesackloch (133, 135) umfasst, das mindestens auf einen Teil des Langlochs (113, 115) ausgerichtet ist;
die Handorthese (100) mit aufblasbarer Blase eine Schraube (140, 141) umfasst, die durch das Langloch (113, 115) hindurch geht und in das Gewindesackloch (133, 135) eintritt;
ein erstes Ende (114, 116) des Langlochs (113, 115) geeignet ist, um in einen Schaft der Schraube (140, 141) einzugreifen, wenn sich der Blasenträger (130) in der radialen Endschwenkposition im Verhältnis zu dem palmaren Schienenabschnitt (111) befindet; und
ein zweites, gegenüberliegendes Ende (117, 118) des Langlochs (113, 115) geeignet ist, um in den Schaft einzugreifen, wenn sich der Blasenträger (130) in der ulnaren Endschwenkposition im Verhältnis zu dem palmaren Schienenabschnitt (111) befindet.

4. Handorthese mit aufblasbarer Blase nach Anspruch 3, **dadurch gekennzeichnet, dass**
der palmare Schienenabschnitt (111) umfasst:
ein erstes Langloch (113), das entlang der radialen-ulnaren Achse (1) auf einer radialen Seite im Verhältnis zu dem Schwenkpunkt (3) angeordnet ist; und
ein zweites Langloch (115), das entlang der radialen-ulnaren Achse (1) auf einer ulnaren Seite im Verhältnis zum Schwenkpunkt (3) angeordnet ist;
der Blasenträger (130) umfasst:
ein erstes Gewindesackloch (133), das mindestens auf einen Teil des ersten Langlochs (113) ausgerichtet ist; und
ein zweites Gewindesackloch (135), das mindestens auf einen Teil des zweiten Langlochs (115) ausgerichtet ist;
die Handorthese (100) mit aufblasbarer Blase umfasst:
eine erste Schraube (140), die durch das erste Langloch (113) hindurch geht und in das erste Gewindesackloch (133) eintritt; und
eine zweite Schraube (141), die durch das zweite Langloch (133) hindurch geht und in das zweite Gewindesackloch (135) eintritt;
ein erstes Ende (114) des ersten Langlochs (113) geeignet ist, um in einen Schaft der ersten Schraube (140) einzugreifen, und ein zweites Ende (118) des zweiten Langlochs (115) geeignet ist, um in einen Schaft der zweiten Schraube (141) einzugreifen, wenn sich der Blasenträger (130) in der radialen Endschwenkposition im Verhältnis zu dem palmaren Schienenabschnitt (111) befindet; und
ein zweites, gegenüberliegendes Ende (117) des ersten Langlochs (113) geeignet ist, um in den Schaft der ersten Schraube (140) einzugreifen, und ein erstes, gegenüberliegendes Ende (116) des zweiten Langlochs (115) geeignet ist, um in den Schaft der zweiten Schraube (141) einzugreifen, wenn sich der Blasenträger (130) in der ulnaren Endschwenkposition im Verhältnis zu dem palmaren Schienenabschnitt (111) befindet.

5. Handorthese mit aufblasbarer Blase nach einem der Ansprüche 1 bis 4, **gekennzeichnet durch** ein Gleitblech (150), das zwischen dem palmaren Schienenabschnitt (111) und dem Blasenträger (130) angeordnet ist, um das Verschwenken des Blasenträgers (130) im Verhältnis zu dem palmaren Schienenabschnitt (111) zu ermöglichen.

6. Handorthese mit aufblasbarer Blase nach einem der Ansprüche 1 bis 5, **dadurch gekennzeichnet, dass**
der palmare Schienenabschnitt (111) ein Durchgangsloch (119) umfasst, das sich durch eine Dicke des palmaren Schienenabschnitts (111) hindurch erstreckt;
der Blasenträger (130) ein Gewindesackloch (139) umfasst, das auf das Durchgangsloch (119) ausgerichtet ist; und
eine Handorthese (100) mit aufblasbarer Blase ferner eine Schraube (142) umfasst, die durch das Durchgangsloch (119) hindurch geht und in das Gewindesackloch (139) eintritt, um den Blasenträger (130) mit dem palmaren Schienenabschnitt (111) schwenkbar zu verbinden.

7. Handorthese mit aufblasbarer Blase nach einem der Ansprüche 1 bis 6, **dadurch gekennzeichnet, dass** der Handwurzel- und Unterarm-Schienenabschnitt (112) umfasst:
einen Unterarmteil (112B), der auf den Unterarm auszurichten ist; und
einen Handwurzelteil (112A), der den Unterarmteil (112B) mit dem palmaren Schienenabschnitt (111) verbindet, wobei der Handwurzelteil (112A) mindestens in einem Teil verformbar ist, der geeignet ist, um auf das Handgelenk der Hand ausgerichtet zu sein.

8. Handorthese mit aufblasbarer Blase nach einem der Ansprüche 1 bis 7, **gekennzeichnet durch** einen Stoffverband (160), der eine Innenkammer aufweist, die geeignet ist, um die Schiene (110), den Blasenträger (130) und die aufblasbare Blase (120) aufzunehmen, wobei der Stoffverband (160) mehrere Verschlussbänder (161, 162, 163) umfasst, die entlang einer distalen-proximalen Achse (2) der Handorthese (100) mit aufblasbarer Blase angeordnet sind, wobei mindestens ein Verschlussband (161) in einem Teil des Stoffverbands (160) angeordnet ist, der auf den palmaren Schienenabschnitt (111) ausgerichtet ist, wenn die Schiene (110) in der Innenkammer des Stoffverbands (160) aufgenommen ist, und mindestens ein Verschlussband (162, 163) umfasst, das in einem Teil des Stoffverbands (160) angeordnet ist, der auf den Handwurzel- und Unterarm-Schienenabschnitt (112) ausgerichtet ist, wenn die Schiene (110) in der Innenkammer des Stoffverbands (160) aufgenommen ist, wobei die mehreren Verschlussbänder (161, 162, 163) angeordnet sind, um die Handorthese (100) mit aufblasbarer Blase an der Hand und dem Unterarm zu fixieren.

## Revendications

1. Orthèse de main à vessie gonflable (100) comprenant :
une attelle (110) comprenant :
une section d'attelle palmaire (111) adaptée pour être alignée avec au moins une partie d'une paume d'une main d'un sujet humain ; et
une section d'attelle de poignet et d'avant-bras (112) d'un seul tenant avec ou reliée à ladite section d'attelle palmaire (111), ladite section d'attelle de poignet et d'avant-bras (112) s'étendant à partir de ladite section d'attelle palmaire (111) sur une distance correspondant à au moins une partie d'un avant-bras dudit sujet humain ; et
une vessie gonflable (120) adaptée pour être alignée avec des articulations métacarpo-phalangiennes (MCP) des doigts de ladite main, ladite vessie gonflable (120) étant gonflable par introduction d'un fluide à l'intérieur d'une chambre intérieure de ladite vessie gonflable (120),
un support de vessie (130) relié à ladite section d'attelle palmaire (111) et comprenant une surface de liaison de vessie (131) opposée à une surface (132) dudit support de vessie (130) faisant face à ladite section d'attelle palmaire (111) ; et
ladite vessie gonflable (120) est disposée sur ladite surface de liaison de vessie (131),
**caractérisée par le fait que** le support de vessie est relié de manière pivotante à la section d'attelle palmaire.

2. Orthèse de main à vessie gonflable selon la revendication 1, **caractérisée par le fait que** ledit support de vessie (130) est pivotante par rapport à ladite section d'attelle palmaire (111) autour d'un point de pivotement (3) entre une position de pivotement d'extrémité radiale et une positon de pivotement d'extrémité ulnaire.

3. Orthèse de main à vessie gonflable selon la revendication 2, **caractérisée par le fait que** :
ladite section d'attelle palmaire (111) comprend une fente (113, 115) ménagée le long d'un axe radial-ulnaire (1) passant par ledit point de pivotement (3) ;
ledit support de vessie (130) comprend un trou borgne fileté (133, 135) aligné avec au moins une partie de ladite fente (113, 115) ;
ladite orthèse de main à vessie gonflable (100) comprend une vis (140, 141) traversant ladite fente (113, 115) et entrant dans ledit trou borgne fileté (133, 135) ;
une première extrémité (114, 116) de ladite fente (113, 115) est adaptée pour engager une tige de ladite vis (140, 141) lorsque ledit support de vessie (130) est dans ladite position de pivotement d'extrémité radiale par rapport à ladite section d'attelle palmaire (111) ; et
une seconde extrémité opposée (117, 118) de ladite fente (113, 115) est adaptée pour engager ladite tige lorsque ledit support de vessie (130) est dans ladite position de pivotement d'extrémité ulnaire par rapport à ladite section d'attelle palmaire (111).

4. Orthèse de main à vessie gonflable selon la revendication 3, **caractérisée par le fait que**
ladite section d'attelle palmaire (111) comprend :
une première fente (113) ménagée le long dudit axe radial-ulnaire (1) sur un côté radial par rapport audit point de pivotement (3) ; et
une seconde fente (115) ménagée le long dudit axe radial-ulnaire (1) sur un côté ulnaire par rapport audit point de pivotement (3) ;
ledit support de vessie (130) comprend :
un premier trou borgne fileté (133) aligné avec au moins une partie de ladite première fente (113) ; et
un second trou borgne fileté (135) aligné avec au moins une partie de ladite seconde fente (115) ;
ladite orthèse de main à vessie gonflable (100) comprend :
une première vis (140) traversant ladite première fente (113) et entrant dans ledit premier trou borgne fileté (133) ; et
une seconde vis (141) traversant ladite seconde fente (133) et entrant dans ledit second trou borgne fileté (135) ;
une première extrémité (114) de ladite première fente (113) est adaptée pour engager une tige de ladite première vis (140) et une seconde extrémité (118) de ladite seconde fente (115) est adaptée pour engager une tige de ladite seconde vis (141) lorsque ledit support de vessie (130) est dans ladite position de pivotement d'extrémité radiale par rapport à ladite section d'attelle palmaire (111) ; et
une seconde extrémité opposée (117) de ladite première fente (113) est adaptée pour engager ladite tige de ladite première vis (140) et une première extrémité opposée (116) de ladite seconde fente (115) est adaptée pour engager ladite tige de ladite seconde vis (141) lorsque ledit support de vessie (130) est dans ladite position de pivotement d'extrémité ulnaire par rapport à ladite section d'attelle palmaire (111).

5. Orthèse de main à vessie gonflable selon l'une quelconque des revendications 1 à 4, **caractérisée par** une feuille coulissante (150) disposée entre ladite section d'attelle palmaire (111) et ledit support de vessie (130) pour faciliter un pivotement dudit support de vessie (130) par rapport à ladite section d'attelle palmaire (111).

6. Orthèse de main à vessie gonflable selon l'une quelconque des revendications 1 à 5, **caractérisée par le fait que** :
ladite section d'attelle palmaire (111) comprend un trou traversant (119) s'étendant à travers une épaisseur de ladite section d'attelle palmaire (111) ;
ledit support de vessie (130) comprend un trou borgne fileté (139) aligné avec ledit trou traversant (119) ; et
ladite orthèse de main à vessie gonflable (100) comprend en outre une vis (142) traversant ledit trou traversant (119) et entrant dans ledit trou borgne fileté (139) pour relier de manière pivotante ledit support de vessie (130) à ladite section d'attelle palmaire (111).

7. Orthèse de main à vessie gonflable selon l'une quelconque des revendications 1 à 6, **caractérisée par le fait que** ladite section d'attelle de poignet et d'avant-bras (112) comprend :
une partie avant-bras (112B) destinée à être alignée avec ledit avant-bras ; et
une partie poignet (112A) interconnectant ladite partie avant-bras (112B) et ladite section d'attelle palmaire (111), ladite partie poignet (112A) étant déformable au moins dans une partie adaptée pour être alignée avec l'articulation de poignet de ladite main.

8. Orthèse de main à vessie gonflable selon l'une quelconque des revendications 1 à 7, **caractérisée par** un pansement textile (160) ayant une chambre intérieure adaptée pour recevoir ladite attelle (110), ledit support de vessie (130) et ladite vessie gonflable (120), ledit pansement textile (160) comprenant de multiples bandes de fermeture (161, 162, 163) disposées le long d'un axe distal-proximal (2) de ladite orthèse de main à vessie gonflable (100) avec au moins une bande de fermeture (161) disposée à une partie dudit pansement textile (160) alignée avec ladite section d'attelle palmaire (111) lorsque ladite attelle (110) est reçue dans ladite chambre intérieure dudit pansement textile (160) et au moins une bande de fermeture (162, 163) disposée à une partie dudit pansement textile (160) alignée avec ladite section d'attelle de poignet et d'avant-bras (112) lorsque ladite attelle (110) est reçue dans ladite chambre intérieure dudit pansement textile (160), lesdites multiples bandes de fermeture (161, 162, 163) étant disposées pour fixer ladite orthèse de main à vessie gonflable (100) à ladite main et audit avant-bras.
